Europäisches Patentamt

European Patent Office

Office européen des brevets

(11) Publication number: **0 230 556 B1**

# EUROPEAN PATENT SPECIFICATION

(45) Date of publication of patent specification: **17.06.92**

(21) Application number: **86116441.6**

(22) Date of filing: **26.11.86**

A request pursuant to Rule 88 EPC for addition of text to claim 3 has been filed.

(51) Int. Cl.⁵: **A61K  35/74**, A61K 39/39, C07K 13/00, //C12P21/02

(54) **Fraction obtained from Listeria monocytogenes having therapeutical activity, process for its preparation and pharmaceutical compositions containing it.**

(30) Priority: **26.11.85 IT 2298985**

(43) Date of publication of application: **05.08.87 Bulletin  87/32**

(45) Publication of the grant of the patent: **17.06.92 Bulletin  92/25**

(84) Designated Contracting States: **AT BE CH DE ES FR GB GR IT LI LU NL SE**

(56) References cited:
FR-A- 2 164 510
FR-A- 2 293 213
FR-A- 2 480 604

**CANCER IMMUNOL IMMUNOTHER, vol. 17, no. 1, 1984, page 38, Springer-Verlag; D. IANNELLO et al.: "Inhibition of normal rat macrophage functions by soluble tumor products effect of systemic treatment with bacterial immunomodulators"**

(73) Proprietor: **SIBAR Srl Sant'Andrea delle Fratte S. Sisto I-06080 Perugia(IT)**

(72) Inventor: **Mastroeni, Pasquale Nuova Panoramica d.Stretto Valle dei Platani 4 I-98100 Messina(IT)**

(74) Representative: **Tiedtke, Harro, Dipl.-Ing. et al Patentanwaltsbüro Tiedtke-Bühling-Kinne-Grupe-Pellmann-Grams-Struif-Winter-Roth Bavariaring 4 W-8000 München 2(DE)**

CHEMICAL ABSTRACTS, vol. 97, no. 23, 6th December 1982, page 454, abstract no. 196828w, Columbus, Ohio, US; & JP-A-82 139 019 (Y. YAMAMURA et al.) 27-08-1982

CAN. J. MICROBIOL., vol. 28, no. 12, 1982, pages 1373-1381, National Research Council of Canada; T.V. OTOKUNEFOR et al.: "Immunosuppression, nonspecific B-cell activation, and mitogenic activity associated with a high molecular weight component from Listeria monocytogenes"

INFECTION AND IMMUNITY, vol. 39, no. 3, March 1983, pages 1114-1121, American Society for Microbiology; N.W. HETHER et al.: "Chemical composition and biological functions of Listeria monocytogenes cell wall preparations"

CELLULAR IMMUNOLOGY, vol. 88, no. 1, 1984, pages 29-40, Academic Press, Inc.; A. NAKANE et al.: "The significance of alpha/beta interferons and gamma interferon produced in mice infected with Listeria monocytogenes"

CAN. J. MICROBIOL., vol. 28, no. 12, 1982, pages 1373-1381 National Research Council of Canada; T.V. OTOKUNEFOR et al.: "Immunosuppression, nonspecific B-cell activation, and mitogenic activity associated with a high molecular weight component from Listeria moncytogenes"

EP 0 230 556 B1

## Description

The present invention relates to a novel therapeutically active fraction, isolated from the fermentation broth of Listeria monocytogenes strain L 79, to the related process of preparation and to the pharmaceutical compositions containing this fraction as active ingredient.

The mononuclear phagocytary system is an important element of the immunitary defences of the host against neoplasiae (D.O. Adams and R. Snyderman, J. Natl. Cancer Inst., 1979, 16, 1341; J.B. Hibbs, H.A. Chapman, and J.B. Weinberg, J. Reticuloendothel., 1978, 24, 549; D.S. Nelson, E.K. Hopper and M. Nelson, Handbook of Cancer Immunology Vol. I, H. Waters ed., p. 107, Garland Publishing Inc. New York, 1979).

The macrophages may exercise a relevant cytotoxic activity against the tumoral cells, in vitro, if an activation takes place, which can originate from several biological stimuli (R. Keller, Immunobiology of the Macrophage, D.S. Nelson ed., p. 487, Academic press, New York, 1976; L.P. Ruco and M.S. Meltze, Cell. Immunol., 41, 35, 1978).

Several microorganisms, among which Mycobacterium bovis (R.C. Bast, B. Zbar, T. Borsos and al., N. Engl. J. Med., 290-1413-1420; 1458-1469, 1974), Corynebacterium parvum (G. Mathé, P. Puillart and L. Schwarzenberg., Natl. Cancer Inst. Monogr., 35, 361, 1972; L. Israel and B. Halpern, Nouv. Presse Med., 1, 19, 1972), Corynebacterium granulosum (G. Mathé, ibid) and Listeria monocytogenes (S. Youdim, M. Moser and O. Stutman, J. Natl. Cancer Inst., 52, 193, 1974) show remarkable effects of activation of the normal or depressed macrophages.

Moreover, R.C. et al (J. Natl. Cancer Inst., 54 (3), 749, 1975), described how several microorganisms, among which Listeria monocytogenes, stimulate the inmunodefensive response of the host and may inhibit the growth of the tumor.

As a matter of fact, substances of bacterial origin and having immunostimulating properties where already previously isolated from microorganisms of the family of the Corynebacterium, these substances being insoluble in aqueous medium and poorly chemically characterized and being consequently of difficult industrial and therapeutical use.

As an example of the state of the art prior to the present invention there can be cited;

- A.R. Prevot, B.N. Halpern, F. Biozzi, C. Stiffel, D. Mouton, J.C. Morand, Y. Bouthillier and C. Decreusefond (C.R. Acad. Sci., Paris, 1963, 257 series D, 13) described the immunostimulating and anti-tumoral properties of some microorganisms of the Corynebacterium anaerobic species.

A number of subsequent studies were oriented towards the isolation of the active fraction among which, D. Migliore-Samour and P. Jolles (Febs Letters, 1972, 25 (2), 301) disclosed the isolation of a hydrosoluble fraction capable of maintaining the adjuvating properties of the enteric microorganism.

On the other side, P. Lallonette, B. Bizzini and M. Raynaud (N.G. M. 1975, 25, 13) by using the process of Migliore-Samour and Jolles in the fractionating of Corynebacterium granulosum, found that the adjuvating and immunostimulating activity was associated to an insoluble fraction (bearing the abbreviation P 40) and not to the hydrosoluble fraction (bearing the abbreviation S 70).

Such an insoluble fraction characterized from the chemical and biological point of view by B. Bizzini, B. Maro and J. Lallonette (in Medicine et Maladies infectieuses, 78, n. 9 Vol. VIII, Pages 408-414) revealed a particularly interesting therapeutical activity with respect to the tumor P 815 in the mouse.

Moreover, P. Lallonette, B. Maro, A. Schwartz and B. Bizzini (C. R. Acad. Sci., Paris 1976), still for the insoluble fraction P 40, revealed the capacity of restoring and increasing the immunogenic properties of forming anti-bodies anti-red cells of ram in the depressed mouse by injection of cyclophosphamide.

Later on, in order to have a complete picture of the prior art, T. Metianu and B. Bizzini (French Patent Application No. 8008976, 1980) found a novel fraction extracted from aerobic bacteria and endowed with anti-tumoral, anti-bacterial and interferon production inducing properties, and the related preparation method.

On the other hand, said fraction too, obtained from Corynebacterium catarrhalis, is also insoluble in aqueous medium.

In view of the already acquired knowledge of the possible influence of Listeria Monocytogenes in the immunodefensive processes and on the basis of the hypothesis that the same microorganism may contain or induce the production of active principles capable of activating the immunitary system, the main purpose of the present invention has been just the search and isolation of such an active principle which at the same time would be endowed with well defined chemical and chemico-physical properties and would be reproducible on an industrial scale.

Another purpose of the present invention is that of isolating an active principle such as that indicated in the preceding paragraph which is also soluble in an aqueous medium with the attendant relevent advantages of industrial and therapeutical use.

3

As already mentioned, the active fraction forming the subject of the present invention, (which is hereinafter indicated for easy use as LM84) has been obtained from a strain of Listeria Monocytogenes L 79 isolated as to the type at the Institute of Microbiology of the University of Messina and corresponding to the following specifications: Listeria monocytogenes strain L 79 isolated from pathological material and isolated as to the type at the Institute of Microbiology of Messina.

- short rod, Gram-positive, asporigen, aerobical or microaerobical; at 20-25°C, seldom at 37°C, shows a tumultuous rotatory mobility which is fully characteristic;

```
- katalase                        +

- metachromatic granules          -

- haemolysis                      + (beta)

- production of acid from:

  glucose                         +

  lactose                         -



  maltose                           +

  mannitol                          -

  solicine                          +

  saccharose                        -

  threolose                         +

  xylose                            -

  dulcitol                          -

- UVP                               +

- hydrolysis of esculin             +

- nitrate reduction                 -

- gelatin liquefaction              -

- urease                            -

- hydrolysis of arginine            -

- Simmons citrate                   -

- phenylalanine deaminase           -

- carrier of the prophage LM 79.
```

There is firstly described the method of preparation of the subject active fractions.

Culture a Medium:

| Bacto-Nutrient broth dehydrated (Difco)® | 8 g |
| distilled water | 1000 ml |

4

The pH is adjusted to 7.2 and the medium is sterilized for 15 minutes at 120°C; there is added the 30% (w/v) glucose solution, sterile, at the final concentration of 0.2%.

Culture

The lyophilic strain of Listeria monocytogenes L 79 is taken in 2 ml of culture medium. The bacterial suspension is transferred in 4 tubes containing 20 ml of culture medium (0.5 ml/tube).

When the bacteria started active multiplication, the content of each tube is transferred to an Erlenmeyer flask containing 500 ml of culture medium.

The culture developed in each flask is used for the seeding of a 5 ℓ big flask (usually a 15 hours culture is used as inoculum).

The culture is interrupted after 48 hours; at which time the bacteria are deposited at the bottom of the big flask.

The supernatant liquid is removed by siphoning; the bacteria are collected by centrifugation and then suspended again in distilled water, decanted again and washed a second time always with distilled water and subsequently centrifuged.

Preparation of the fraction LM 84

On the washed bacteria (about 1.9 g/ℓ of culture) a delipidation is carried out with a Soxhlet equipment by carrying out alternatively the following treatments:

a) 12 hours with ether-ethanol (1:1), then for 12 hours with chloroform and then for a further 12 hours always with chloroform mixed with methanol (2:2).

b) three subsequent treatments with acetone, each having a duration of 12 hours.

The delipidated bacteria are disintegrated in a mechanical Potter homogeneizer (10 times for 1 minute at maximum speed; cooling in ice).

The suspension of disintegrated bacteria is maintained under constant stirring for 5 hours at room temperature before being centrifuged at 2000 x g in order to eliminate the intact bacteria.

The supernatant is heated to 80°C before being adjusted at the concentratation of 40% saturation by addition of a saturated solution of ammonium sulphate (or, alternatively, with mono- and di-potassium phosphate buffer 1.25 M).

After one night at 4°C, the thus formed precipitated is collected by centrifugation at $10000 \times g$.

The precipitate is suspended again in distilled water and dialized against distilled water until the presence of ammonium sulphate (or alternatively of potassium phosphate) is no longer detectable.

The obtained fraction (about 6 mg/g of delipidated bacteria) corresponding to a fraction insoluble in aqueous medium, is lyophilized.

After lyophilization it is solubilized in the presence of urea at the concentration 6M (or alternatively in the presence of sodium hydrogen carbonate).

In the case in which the solubilization is carried out with urea, a subsequent dialysis against distilled water is carried out until all traces of urea are totally eliminated.

The fraction remains soluble in aqueous medium even after elimination of the solubilizing medium.

Then the purification of the active substance is carried out by chromatography in a column against Tris buffer on Sepharose 6 B or alternatively on Ultragel Ac A 34.

The results of the analyses as thereinafter reported represent the average values obtained on a total number of 10 batches obtained with the process of the extraction and purification as above described.

Protein content

The dosage, carried out according to the method of Lowry, gave a protein content of 0.75 ± 0.1 mg/mg of active fraction LM 84.

Moisture content

up to 10%

Ashes

from 0.05 to 0.08 mg of ashes/mg of LM 84 (dried weight).

## Neutral sugars (anthrone method)

from 0.05 to 2% of neutral sugars/mg of LM 84.

## Hexosamines (Elson Morgan method)

from 0.6 to 1.8% of hexosamines/mg of LM 84.

## Molecular weight

The molecular weight has been determined by column chromatography on Ultragel AcA 34 set with the following reference substances: IgG, bovine seroalbumin, egg albumin, myoglobin (kit of Pharmacia).

The fraction is eluted in form of a unique peak slightly asymmetrical on the descending side.

The molecular weight is between 290.000 and 330.000 Daltons (average value: 300.000 Daltons)

| Determination of the aminoacidic composition by aminoacid analyzer | | | |
|---|---|---|---|
| Amminoacid | $\mu$g | % | numer of AA residues per molecule |
| Asp | 57.76 | 6.49 | 155 |
| Thr | 21.32 | 2.40 | 64 |
| Ser | 22.49 | 2.53 | 77 |
| Glu | 193.45 | 21.73 | 470 |
| Pro | 36.25 | 4.07 | 113 |
| Gly | 18.69 | 2.10 | 89 |
| Ala | 58.35 | 6.56 | 234 |
| Val | 26.59 | 2.99 | 81 |
| Cys[+] | - | - | - |
| Met | 5.97 | 0.67 | 14 |
| Ile | 29.78 | 3.35 | 81 |
| Leu | 63.60 | 7.15 | 174 |
| Tyr | 17.21 | 1.93 | 34 |
| Phe | 12.06 | 1.36 | 26 |
| Lys | 93.20 | 10.47 | 228 |
| His | 11.15 | 1.25 | 26 |
| Arg | 39.25 | 4.41 | 81 |
| DAP | 153.00 | 17.19 | 288 |
| Total | 860.12 | 96.65 | 2237 |
| Cys[+] has been determined in form a CM-Cys. | | | |

The determination of the aminoacid composition has been carried out in a three fold way by means of an automatic analyzer Beckman model Multichrom B 4255 according to the method of Spackman (Anal. Chem. 1958, 30, 1190) on an acid hydrolysate of 24 hours.

The methionine has been determined according to the method of Moore (J. Biol. Chem; 1963, 238, 235).

Tryptophan has been determined according to the colorimetric method of Gaitonde and Dovey (Biochem. J., 1970, 117, 907).

## Analysis of the pharmacological and biological properties of fraction LM 84

## Determination of the toxicity properties

The administration of the substance in the mouse by subcutaneous, intramuscular and intravenous route, up to dosages of 1 mg/mouse (corresponding to 50 mg/kg of body weight) do not induce any toxic phenomena.

## Analysis of the effects on the growth of tumoral mass and inhibiting the metastasis forming

T8 of Guérin, an atypical transplantable epithelioma has been used. The tumor has been transplated in rats, Norvegicus strain, every 20 days in the dorsal subcutaneous area; in a second experiment for the control of the influence of the metastasis formation, the transplantation was carried out in the same area daily.

The fraction LM 84 has been administered in single dose by intravenous route (200 μg/rat) three days after the transplantation of the tumor. As demonstrated by the growth curve of the tumor T8 Guérin in the rat (both treated and non treated), the growth of the tumor T8 is significatively inhibited by a single intravenous administration of the compound.

The development of the metastasis was fully inhibited by the treatment with the compound; the average weight of the metastasis in the non-treated animals 20 days after the transplantation, was 3.196 ± 164 mg.

Phagocytosis and intracellular killing of Candida albicans

For the test of phagocytosis and of killing of Candida albicans by the peritoneal macrophages the micromethod of Smith and Rommel was used (J. Immunol. Methods, 1977, 17, 241).

The phagocytic activity of the peritoneal macrophages of the non treated, tumor carrying rats, was remarkably depressed with respect to the control animals; the intracellular killing of C. albicans in said animals was unchanged.

On the contrary, in the following table 1, it is demonstrated that the phagocytic activity of the macrophages of tumor carrying rats and treated with the fraction LM 84 is restored to higher values with respect to those of the control macrophages.

In the same table it can be noted that the intracellular killing activity is significatively influenced by the treatment with the fraction LM 84 but at a lower degree of the phagocytic activity (p < 0.001).

TABLE 1

Effect of the intravenous administration (only one dose of 200 μg/rat) of the fraction LM 84 on the phagocytosis and on intracellular killing of Candida Albicans in the tumor carrying rat.

|  | Phagocytosis % | Killing % |
|---|---|---|
| Control rat | $60 \pm 6^{a}$ | $21 \pm 4$ |
| Tumor carrying rat, non treated | $30 \pm 5$ | $18 \pm 2$ |
| Tumor carrying rat, treated with LM 84 | $94 \pm 9^{a}$ | $24 \pm 2.4^{b}$ |

The results are expressed as the average of 5 experiments, ± the standard error;

a= significant difference (p < 0.0001) with respect to the tumor carrying rats non treated;

b= significant difference (p < 0.001) with respect to the tumor carrying rats non treated.

Effect of the treatment with fraction LM 84 on the chemotactic response of the peritoneal macrophages.

The serum of rat activated with endo-toxin has been prepared by incubation of normal serum with 1 mg/ml of endotoxin from Escherichia coli. The method used has been that disclosed by Maderazo and Waronick (A modified micropore filter assay of human granulocyte chemotaxis, in: Quie PG, Gallin JL eds.,

Leukocyte chemotaxis, Raven Press, New York, 1978, p. 43).

All the experiments have been carried out in a three fold manner and the avarage chemotactic index (CI) and the migration distance have been calculated.

The chemotactic response of peritoneal cells of tumor carrying rats was remarkably depressed with respect with the control macrophages.

Both the chemotatic index and the average distance as $\mu$ of migration of cells were significatively reduced in the tumor carrying animals with respect to the control values.

Table 2 demonstrates that the chemotactic response of the peritoneal cells obtained in the animals treated with fraction LM 84 is significatively developed ($p < 0.0001$).

TABLE 2

| Effect of the intravenous administration (200 $\mu$g/rat) of fraction LM 84 on the chemotactic response of the peritoneal macrophages in the tumor carrying rats. | | |
| --- | --- | --- |
| | Chemotactic index | Migration front |
| Control rats | $45.25 \pm 5.2^a$ | $118 \pm 9^b$ |
| Tumor carrying rat, non treated | $31.50 \pm 2.8$ | $58 \pm 6.1$ |
| Thumor carrying rat treated with LM 84 | $50.00 \pm 5.0^b$ | $122 \pm 10.2^b$ |

a = significant difference ($p < 0.001$) with respect to the tumor carrying rats non treated;
b = significant difference ($p < 0.0001$) with respect to the tumor carrying rats non treated.

Effect on the treatment with fraction LM 84 on the intrinsic activity anti-HSV-1 of the peritoneal macrophages.

The effect has been tested with the method described by Bonina at al. (Infect Immun., 1983, 39, 575).

The intrinsic activity anti-HSV-I of the peritoneal macrophages has been evaluated, for several groups of rats, by analyzing the development curves of HSV-I.

The following table III confirms that the production of HSV-I after 24 hours of incubation was higher in the macrophages obtained from tumor carrying rats ($p < 0.002$) than in the macrophages collected from the control rats or from the tumor carrying rats but treated with the fraction LM 84.

On the contrary no statistical difference exists between the content of HSV-I of the macrophages of tumor carrying rats and treated with LM 84 in comparison with the control macrophages.

8

TABLE III

Intrinsic activity anti HSV-I of macrophages of tumor carrying rats treated with LM 84 in only one dose by intravenous route (200 $\mu$g/rat)

|  | | Production of HSV-I in 24 hours (PFU/ml) | |
|---|---|---|---|
| control rats | MOI 1 | $2 \times 10^5$ | $\pm\ 1 \times 10^2$ |
|  | MOI 10 | $4 \times 10^5$ | $\pm\ 2 \times 10^2$ |
| Non treated tumor carrying rats | MOI 1 | $8 \times 10^6$ | $\pm\ 3 \times 10^3$ |
|  | MOI 10 | $2 \times 10^5$ | $\pm\ 2 \times 10^3$ |
| Tumor carrying rats treated with LM 84 | MOI 1 | $1 \times 10^5$ | $\pm\ 2 \times 10^2$ a |
|  | MOI 10 | $1 \times 10^5$ | $\pm\ 1 \times 10^2$ a |

The results are expressed as the average of the values of 5 experiments.

a= p $<$ 0.002 in comparison with the tumor with the carrying rats non treated.

Effect of the treatment with fraction LM 84 on the extrinsic activity anti-HSV-I of peritoneal macrophages.

By extrinsic activity the mechanism is meant by which the macrophages may inhibit the multipling of virus in permitting neigh bouring cells.

This activity has been tested according to the method disclosed by Wildy et al (Infect Immun. 1982, 37, 40).

The extrinsic activity anti-HSV-I has been expressed in terms of reduction of growth of the virus at the use concentration of arginine (Bonina at al, Virus Res., 1984, 1 501).

As demonstrated by the following table IV the growth of HSV-I as obtained on Vero cells was not statistically different in the case of cells incubated with peritoneal cells of tumor carrying rats or of control rats.

On the contrary, the growth of HSV-I on Vero cells cultivated together with different concentrations of peritoneal cells of tumor carrying rats treated with the fraction LM 84 was significatively different (p < 0.002; p < 0.001) with respect to the values obtained with macrophages of tumor carrying rats non treated and with macrophages of control rats.

TABLE IV

Extrinsic activity. anti-HSV-I of macrophages of tumor carrying rats treated with LM 84    .

| | Ratio macrophages /Vero cells | Extrinsic activity (a) |
|---|---|---|
| Control rats | 5/1 | $6 \times 10^6 \pm 2 \times 10^2$ |
| | 10/1 | $2 \times 10^6 \pm 2 \times 10^2$ |
| Tumor carrying rats non treated | 5/1 | $9 \times 10^6 \pm 1 \times 10^2$ |
| | 10/1 | $6 \times 10^6 \pm 3 \times 10^2$ |
| Tumor carrying rats treated with LM 84 | 5/1 | $1 \times 10^5 \pm 6 \times 10^3 b$ |
| | 10/1 | $1 \times 10^4 \pm 2 \times 10^2 c$ |
| Vero cells | | $2 \times 10^7 \pm 6 \times 10^2$ |

a= the results are expressed as growth of the virus after 24 hours incubation: average of 4 experiments;

b= significant difference (p $<$ 0.002) with respect to non treated carrier

c= significant difference (p $<$ 0.001) with respect to the values of non treated carriers.

Response of the intravenous treatment of LM 84 (200 $\mu$g/kg/per day for 7 days) to the administration of ram red cells (GRM)

The haemoagglutinins have been dosed in the Swiss mouse according to the method of Biozzi et al. (Ann. Inst. Pasteur, 1966, 110, suppl. 3, 7-32); the reading has been carried out according to the technique on microplate (Raynaud et al. Ann. Inst. Pasteur, 1972, 122, 695).

The ram red cells have been administered at the dose of $5 \times 10^6$ cells. From the variation of haemoagglutinating concentration as a function of the time it can be noted, in the long run, that the haemoagglutinating concentration is maintained for the control animals on very low values.

On the contrary in the animals treated with the fraction LM 84, the concentration is quickly increased achieving the maximum value at about 1 week from the injection of GRM.

Determination of the interferon inducing activity.

The experiments have been carried out in Swiss mice of the weight of 20g; the product has been administered at dose of 200 $\mu$g by intravenous route with a volume of 0.5 ml, whereas the control animals were administered with an equal volume of physiological solution.

The blood abstraction for the determination of the concentration were carried out at 1,2,4,8, 24 and 48

EP 0 230 556 B1

hours from the administration of the compound.

The determination of interferon has been carried out according to the method of inhibition of the cytopathic effect by using cells lines L929 and the virus of vescicular stomatitis as competitive virus (Metianu et al. Comp. Immun. Microbiol. Infect. Dis, 1981, 4, 24).

The concentration of interferon was adjusted as units with reference to a standard serum.

The results are shown in the following table V:

TABLE V

| Interferon inducing activity of intravenous treatment with LM 84 | | | | | | |
|---|---|---|---|---|---|---|
| | Interferon in international units Hours | | | | | |
| | 1 | 2 | 4 | 8 | 24 | 48 |
| LM 84 (200 $\mu$giv) carrier | 100 3 | 400 3 | 400 3 | 300 3 | 200 3 | 100 3 |

From the above tests and having a specific purpose and validity and which are not generical ones it can be concluded that the fraction abbreviated as LM 84 and forming the subject of the present invention is capable of:

- inhibiting the growth of the tumoral mass and the metastasis formation;
- having immunostimulating effect (stimulation of the phagocytic activity); increase of the killing (activation of the microbicidal activity of the macrophage); restoring of the anti-viral activity of macrophages;
- inducing the interferon production;
- enhacing the anti-body production.

Consequently it can be therapeutically used in the oncogeuic forms, in the infections and generally in all pathological forms involving a compromising the immunodefensive system.

From the experimental condition and from the properties of the substance it is also possible to realize the administration routes (intramuscolar and intravenous) and the dosages (from $5 \cdot 10^{-5}$ to $10^{-4}$ g (50 to 400 $\gamma$) in only one administration or periodical cycles of 1-3 weeckly administrations according to the specific pathology) to be adopted in the clinical use of the substance itself.

## Claims

1. Active fraction, obtained from a culture of Listeria monocytogenes strain L 79, by means of delipidation of the bacteria, their disintegration, precipitation by means of an agent selected among ammonium sulphate and phosphate buffer, dialysis against distilled water, lyophilization of the insoluble fraction, solubilization in the presence of a compound selected among urea and sodium acid carbonate and lastly chromatographic purification.

2. Active fraction according to claim 1, **characterized by** having the following analytical characteristics:
   - protein content according to Lowry: 0.75 ± 0.1 mg/mg of active fraction;
   - moisture content: up to 10 %;
   - ashes: 0.05-0.08 mg/mg of dry weight of fraction;
   - neutral sugars (anthrone method): 0.5-2 % of neutral sugars/mg of active fraction;
   - hexosamine (Elson-Morgan method): 0.6-1 % of hexosamine/mg of active fraction;
   - molecular weight: between 290.000 and 330.000 Daltons, with an average value of 300.000;

3. Active fraction according to claim 2, **characterized by** having the following aminoacidic composition, referred to a 24 hours hydrolysate:

11

| Determination of the aminoacidic composition by aminoacid analyzer | | | |
|---|---|---|---|
| Amminoacid | $\mu$g | % | numer of AA residues per molecule |
| Asp | 57.76 | 6.49 | 155 |
| Thr | 21.32 | 2.40 | 64 |
| Ser | 22.49 | 2.53 | 77 |
| Clu | 193.45 | 21.73 | 470 |
| Pro | 36.25 | 4.07 | 113 |
| Gly | 18.69 | 2.10 | 89 |
| Ala | 58.35 | 6.56 | 234 |
| Val | 26.59 | 2.99 | 81 |
| Cys[+] | - | - | - |
| Met | 5.97 | 0.67 | 14 |
| Ile | 29.78 | 3.35 | 81 |
| Leu | 63.60 | 7.15 | 174 |
| Tyr | 17.21 | 1.93 | 34 |
| Phe | 12.06 | 1.36 | 26 |
| Lys | 93.20 | 10.47 | 228 |
| His | 11.15 | 1.25 | 26 |
| Arg | 39.25 | 4.41 | 81 |
| DAP | 153.00 | 17.19 | 288 |
| Total | 860.12 | 96.65 | 2237 |

**4.** A process for the preparation of the active fraction according to the preceding claims, characterized by the steps of:
- cultivating Listeria monocytogenes, strain L 79, in a culture medium;
- collecting the resulting bacteria;
- delipidating the bacteria;
- disintegrating the bacteria;
- precipitating an insoluble fraction with an agent selected among ammonium sulphate and phosphate buffer;
- dialyzing against distilled water;
- lyophilizing the insoluble fraction;
- solubilizing the lyophilisate in the presence of an agent selected among urea and sodium acid carbonate, thus giving a fraction soluble in aqueous medium;
- purifying by chromatography.

**5.** A process according to claim 4, characterized in that said Listeria monocytogenes strain L 79 has the following characteristics:
- short Gram-positive rod, asporigen, aerobic or microaerobic; at 20 to 25ºC, seldom at 37ºC, shows a fully peculiar tumultuous rotatory mobility;

```
catalase                        +
metachromatic granules          -
haemolyis                       + (beta)
-acid production from
glucose                         +
lactose
maltose                         +
mannitol                        -
solicine                        +
saccharose                      -


threolose                       +
xylose                          -
dulcitol                        -
UVP                             +
-esculin hydrolysis             +
-nitrate reduction              -
-gelatin liquefaction           -
-urease                         -
-Simmons citrate                -
-phenylalanine deaminase        -
-carrier of LM 79 prophage
```

6. A process according to claim 4, characterized in that said delipidation is carried out in a Soxhlet apparatus, by alternated treatments with
   a) ether-ethanol (1:1) for 12 hours
   b) chloroform for 12 hours
   c) chloroform in admixture with methanol (2:1) for 12 hours;
   d) acetone for three consecutive times, each having a duration of 12 hours.

7. A process according to claim 4, **characterized in that** said disintegration is carried out in a mechanical homogenizer with simultaneous cooling.

8. A process according to claim 1, characterized in that the suspension of disintegrated bacteria is centrifuged to remove the intact bacteria.

9. A process according to claim 8, characterized in that the supernatant layer of the centrifuging step is heated to 80ºC and adjusted to a 40 % saturation concentration by addition of a saturated solution of ammonium sulphate, and is maintained for a night at 4ºC, the precipitate being thereafter recovered by centrifugation.

10. A process according to claim 9, characterized in that instead of ammonium sulphate a 1.25 M buffer of mono- and bi-potassium phosphate is used.

**11.** A process according to the claims 9 or 10, characterized in that the recovered precipitate is suspended in distilled water and depurated from the ammonium sulphate or phosphate buffer, a fraction insoluble in aqueous medium being obtained.

**12.** A process according to claim 11, characterized in that said depuration is carried out by dialysis against distilled water.

**13.** A process according to claim 11, characterized in that said insoluble fraction is lyophilized.

**14.** A process according to claims 4 and 13, characterized in that said lyophilizate is solubilized in the presence of 6 M urea or of sodium acid carbonate, a dialysis against distilled water being thereafter carried out in the case of solubilization in the presence of urea.

**15.** A process according to claims 4 and 14, characterized in that said solubilized fraction is purified by column chromatography.

**16.** Pharmaceutical composition according to the preceding claims characterized by being in a form which is watersoluble and suitable for the administration by intramuscular and intravenous routes after dissolving the lyophilized product in distilled water.

**17.** Pharmaceutical composition according to claim 16, characterized by containing $5 \cdot 10^{-5}$ to $10^{-4}$ g (50 - 100 $\gamma$) of said active fraction.

**18.** Pharmaceutical composition according to claims 16 and 17, for use in the treatment of the oncogenetic forms.

**19.** Pharmaceutical composition according to each of the claims 16 to 18, for use in the treatment of infectious pathologic states of microbial and viral origin.

**20.** Pharmaceutical composition according to each of the claims 16 to 18, for use in the treatment of the pathological states wherein the immunodefensive system is affected.

**Revendications**

**1.** Fraction active obtenue à partir d'une culture d'une souche L 79 de <u>Listeria monocytogenes</u>, au moyen d'une délipidation des bactéries, leur désintégration, la précipitation au moyen d'un agent choisi parmi les tampons au sulfate et phosphate d'ammonium et dialyse par l'eau distillée, lyophilisation de la fraction insoluble, solubilisation en présence d'un composé choisi parmi l'urée et le carbonate acide de sodium, et en dernier lieu, purification par chromatographie.

**2.** Fraction active selon la revendication 1, caractérisée comme présentant les caractéristiques analytiques suivantes :
- Teneur en protéine selon Lowry : 0,75 ± 0,1 mg/mg de la fraction active ;
- Teneur en humidité : Jusqu'à 10% ;
- Cendres : 0,05-0,08 mg/mg de la masse sèche de la fraction active ;
- Sucres neutres (méthode à l'anthrone) : 0,5-2% de sucres neutre /mg de fraction active ;
- Hexosamine (méthode Elson-Morgan): 0,6-1% d'hexosamine par mg de fraction active ;
- Masse moléculaire : entre 290 000 et 330 000 Daltons, avec une valeur moyenne de 300 000.

**3.** Fraction active selon la revendication 2,caractérisée par la composition d'acide aminé suivante, rapportée à un hydrolysat de 24 heures :

| Détermination de la composition des amino-acides par un analyseur d'amino-acides | | | |
|---|---|---|---|
| Amino-acide | µg | % | Nombre de résidus d'AA par molécule |
| Asp | 57,76 | 6,49 | 155 |
| Thr | 21,32 | 2,40 | 64 |
| Ser | 22,49 | 2,53 | 77 |
| Clu | 193,45 | 21,73 | 470 |
| Pro | 36,25 | 4,07 | 113 |
| Gly | 18,69 | 2,10 | 89 |
| Ala | 58,35 | 6,56 | 234 |
| Val | 26,59 | 2,99 | 81 |
| Cys + | - | - | - |
| Met | 5,97 | 0,67 | 14 |
| Ile | 29,78 | 3,35 | 81 |
| Leu | 63,60 | 7,15 | 174 |
| Tyr | 17,21 | 1,93 | 34 |
| Phe | 12,06 | 1,36 | 26 |
| Lys | 93,20 | 10,47 | 228 |
| His | 11,15 | 1,25 | 26 |
| Arg | 39,25 | 4,41 | 81 |
| Dap | 153,00 | 17,19 | 208 |
| Total | 860,12 | 96,65 | 2237 |

**4.** Procédé de préparation de la fraction active selon les revendications précédentes, caractérisé par les étapes suivantes :
- Culture d'une souche L 79 de Listeria monocytogènes, dans un milieu de culture;
- Recueil des bactéries obtenues;
- Délipidation de la bactérie;
- Désintégration de la bactérie;
- Précipitation d'une fraction insoluble par un agent choisi parmi les tampons sulfate et phosphate d'ammonium;
- Dialyse par l'eau distillée;
- Lyophilisation de la fraction insoluble;
- Solubilisation du produit lyophilisé en présence d'un agent choisi parmi l'urée et le carbonate acide de sodium, donnant ainsi une traction soluble en milieu aqueux;
- Purification par chromatographie.

**5.** Procédé selon la revendication 4, caractérisé en ce que la Listeria monocytogenes, souche L 79 possède les caractéristiques suivantes:
- Tige Gram-positive courte, aspérigène, aérobie ou micro-aérobie; entre 20 et 25°C, rare à 37 C, montre une mobilité rotatoire totale particulièrement tumultueuse ;

| | |
|---|---|
| Catalase | + |
| Granules métachromatiques | − |
| Hémolyse | + (béta) |
| Production d'acide à partir de | |
| Glucose | + |
| Lactose | |
| Maltose | + |
| Mannitol | − |
| Solicine | + |
| Saccharose | − |
| Thréolose | + |
| Xylose | − |
| Dulcitol | − |
| UVP | + |
| − Hydrolyse d'esculine | − |
| − Réduction de nitrate | − |
| − Liquéfaction de gélatine | − |
| − Uréase | − |
| − Citrate Simmons | − |
| − Déaminase phénylalanine | − |
| − Prophage de porteur de LM 79 | − |

6. Procédé selon la revendication 4, caractérisé en ce que la délipidation est réalisée dans un appareil de Soxhlet, par des traitements alternés avec:
   a) éther-éthanol (1:1) pendant 12 heures;
   b) chloroforme pendant 12 heures;

EP 0 230 556 B1

c) chloroforme en mélange avec du méthanol (2:1) pendant 12 heures;
d) acétone trois fois de suite, chaque fois pendant 12 heures.

7. Procédé selon la revendication 4, caractérisé en ce que la désintégration est réalisée dans un homogénéiseur mécanique avec refroidissement simultané.

8. Procédé selon la revendication 1, caractérisé en ce que la suspension de bactéries désintégrées est centrifugée pour éliminer les bactéries intactes.

9. Procédé selon la revendication 8, caractérisé en ce que la couche surnageante de l'étape de centrifugation est chauffée à 80°C et ajustée à une concentration à saturation de 40% par addition d'une solution saturée de sulfate d'ammonium et est maintenue pendant une nuit à 4°C, le précipité étant alors récupéré par centrifugation.

10. Procédé selon la revendication 9, caractérisé en ce qu'à la place du sulfate d'ammonium, on utilise un tampon de mono- et diphosphate d'ammonium à 1,25 M.

11. Procédé selon la revendication 9 ou 10, caractérisé en ce que le précipité récupéré est mis en suspension dans l'eau distillée et débarrassé du tampon sulfate ou phophate d'ammonium, une fraction insoluble dans le milieu aqueux étant ainsi obtenue.

12. Procédé selon la revendication 11, caractérisé en ce que l'épuration est réalisée par un dyalise avec de l'eau distillée.

13. Procédé selon la revendication 11, caractérisé en ce que cette fraction insoluble est lyophilisée.

14. Procédé selon les revendications 4 et 13, caractérisée en ce que ce lyophylisat est mis en solution en présence de 6 M d'urée ou de carbonate acide de sodium, une dialyse à l'eau distillée étant ensuite réalisée dans le cas d'une solubilisation en présence d'urée.

15. Procédé selon les revendications 4 et 14, caractérisé en ce que cette fraction mise en solution est purifiée sur une colonne chromatographique.

16. Composition pharmaceutique selon les revendications précédentes, caractérisée en ce qu'elle est sous forme soluble dans l'eau et convient à une administration par voies intramusculaire ou intraveineuse après dissolution dans l'eau du produit lyophilisé.

17. Composition pharmaceutique selon la revendication 16, caractérisée en ce qu'elle contient de $5.10^{-5}$ à $10^{-4}$ g (50 à 100 gamma) de cette fraction active.

18. Composition pharmaceutique selon les revendications 16 et 17, destinée au traitement de formes oncogénétiques.

19. Composition pharmaceutique selon chacune des revendications 16 à 18, destinée au traitement d'états pathologiques infectieux d'origine microbienne et virale.

20. Composition pharmaceutique selon chacune des revendications 16 à 18, destinée au traitement d'états pathologiques dans lesquels le système immunodéfensif est affecté.

**Patentansprüche**

1. Wirksame Fraktion, erhalten aus einer Kultur von Listeria monocytogenes, Stamm L 79, durch Entlipidieren der Bakterien, deren Aufschluß bzw. Desintegration, Fällen mit einem Mittel, das aus Ammoniumsulfat und Phosphatpuffer ausgewählt ist, Dialysieren gegen destilliertes Wasser, Lyophilisieren der unlöslichen Fraktion, Löslichmachen in Gegenwart einer Verbindung, die aus Harnstoff und Natriumhydrogencarbonat ausgewählt ist, und schließlich Reinigen durch Chromatographie.

2. Wirksame Fraktion nach Anspruch 1, dadurch gekennzeichnet, daß sie die folgenden analytischen

17

Eigenschaften hat:

- Proteingehalt nach Lowry: 0,75 ± 0,1 mg/mg der wirksamen Fraktion;
- Feuchtigkeitsgehalt: bis zu 10 %;
- Asche: 0,05 bis 0,08 mg/mg der Trockenmasse der Fraktion;
- Neutrale Zucker (Anthronmethode): 0,5 bis 2 % neutrale Zucker/mg der wirksamen Fraktion;
- Hexosamin (Elson-Morgan-Methode): 0,6 bis 1 % Hexosamin/mg der wirksamen Fraktion;
- Molekulargewicht: zwischen 290,000 und 330,000 Dalton, mit einem Mittelwert von 300,000;

3. Wirksame Fraktion nach Anspruch 2, dadurch gekennzeichnet, daß sie die folgende, auf ein nach 24 h erhaltenes Hydrolysat bezogene Aminosäurezusammensetzung hat:

| Bestimmung der Aminosäurezusammensetzung mit einem Aminosäureanalysiergerät | | | |
|---|---|---|---|
| Aminosäure | μg | % | Zahl der Aminosäurereste je Molekül |
| Asp | 57,76 | 6,49 | 155 |
| Thr | 21,32 | 2,40 | 64 |
| Ser | 22,49 | 2,53 | 77 |
| Glu | 193,45 | 21,73 | 470 |
| Pro | 36,25 | 4,07 | 113 |
| Gly | 18,69 | 2,10 | 89 |
| Ala | 58,35 | 6,56 | 234 |
| Val | 26,59 | 2,99 | 81 |
| Cys$^+$ | - | - | - |
| Met | 5,97 | 0,67 | 14 |
| Ile | 29,78 | 3,35 | 81 |
| Leu | 63,60 | 7,15 | 174 |
| Tyr | 17,21 | 1,93 | 34 |
| Phe | 12,06 | 1,36 | 26 |
| Lys | 93,20 | 10,47 | 228 |
| His | 11,15 | 1,25 | 26 |
| Arg | 39,25 | 4,41 | 81 |
| DAP | 153,00 | 17,19 | 288 |
| Gesamt | 860,12 | 96,65 | 2237 |

4. Verfahren zur Herstellung der wirksamen Fraktion nach den vorhergehenden Ansprüchen, gekennzeichnet durch die folgenden Schritte:

- Kultivieren von Listeria-monocytogenes, Stamm L 79, in einem Nährmedium;
- Sammeln der erhaltenen Bakterien;
- Entlipidieren der Bakterien;
- Aufschluß bzw. Desintegration der Bakterien;
- Fällen einer unlöslichen Fraktion mit einem Mittel, das aus Ammoniumsulfat und Phosphatpuffer ausgewählt ist;
- Dialysieren gegen destilliertes Wasser;
- Lyophilisieren der unlöslichen Fraktion;
- Löslichmachen des Lyophilisats in Gegenwart eines Mittels, das aus Harnstoff und Natriumhydrogencarbonat ausgewählt ist, so daß eine Fraktion erhalten wird, die in einem wäßrigen Medium löslich ist;
- Reinigen durch Chromatographie.

5. Verfahren nach Anspruch 4, dadurch gekennzeichnet, daß Listeria monocytogenes, Stamm L 79, die folgenden Eigenschaften hat:

- kurzes, grampositives Stäbchen, asporogen, aerob oder mikro-aerob;    zeigt bei 20 bis 25 °C, selten bei 37 °C, eine ganz eigenartige, turbulente rotatorische Beweglichkeit;

- Katalase                                    +

- metachromatische Granula      -

- Hämolyse                                 + (beta)

- Säureproduktion aus

  Glucose                                 +

  Lactose                                 -

  Maltose                                 +

  Mannit                                   -

  Solicin                                 +

  Saccharose                           -

  Threolose                             +

  Xylose                                   -

  Dulcit                                   -

  UVP                                         +

- Aesculinhydrolyse                 +

- Nitratreduktion                     -

- Gelatineverflüssigung          -

- Urease                                     -

- Simmons Citrat                       -

- Phenylalanindesaminase       -

- Träger des LM-79-Prophagen


6. Verfahren nach Anspruch 4, dadurch gekennzeichnet, daß das Entlipidieren in einem Soxhlet-Apparat durch abwechselnde Behandlungen

    a) mit Ether-Ethanol (1:1) für eine Dauer von 12 h,
    b) mit Chloroform für eine Dauer von 12 h,
    c) mit Chloroform in Form einer Mischung mit Methanol (2:1) für eine Dauer von 12 h,
    d) dreimal hintereinander mit Aceton für eine Dauer von jeweils 12 h durchgeführt wird.

7. Verfahren nach Anspruch 4, dadurch gekennzeichnet, daß der Aufschluß bzw. die Desintegration in einer mechanischen Homogenisiervorrichtung mit gleichzeitigem Kühlen durchgeführt wird.

8. Verfahren nach Anspruch 1, dadurch gekennzeichnet, daß die Suspension der aufgeschlossenen bzw. desintegrierten Bakterien zentrifugiert wird, um die intakten Bakterien zu entfernen.

9. Verfahren nach Anspruch 8, dadurch gekennzeichnet, daß die überstehende Schicht des Zentrifugierschrittes auf 80 °C erhitzt, durch Zusatz einer gesättigten Lösung von Ammoniumsulfat auf 40 % der Sättigungskonzentration eingestellt und eine Nacht lang bei 4 °C gehalten wird, wobei der Niederschlag danach durch Zentrifugieren gewonnen wird.

10. Verfahren nach Anspruch 9, dadurch gekennzeichnet, daß anstelle von Ammoniumsulfat ein 1,25 m Puffer aus Kaliumdihydrogen- und Dikaliumhydrogenphosphat verwendet wird.

**11.** Verfahren nach Anspruch 9 oder 10, dadurch gekennzeichnet, daß der gewonnene Niederschlag in destilliertem Wasser suspendiert und von dem Ammoniumsulfat oder dem Phosphatpuffer gereinigt wird, wobei eine Fraktion erhalten wird, die in wäßrigem Medium unlöslich ist.

**12.** Verfahren nach Anspruch 11, dadurch gekennzeichnet, daß die Reinigung durch Dialyse gegen destilliertes Wasser durchgeführt wird.

**13.** Verfahren nach Anspruch 11, dadurch gekennzeichnet, daß die unlösliche Fraktion lyophilisiert wird.

**14.** Verfahren nach Ansprüchen 4 und 13, dadurch gekennzeichnet, daß das Lyophilisat in Gegenwart von 6 m Harnstoff oder von Natriumhydrogencarbonat löslich gemacht wird, wobei danach im Fall des Löslichmachens in Gegenwart von Harnstoff eine Dialyse gegen destilliertes Wasser durchgeführt wird.

**15.** Verfahren nach Ansprüchen 4 und 14, dadurch gekennzeichnet, daß die löslich gemachte Fraktion durch Säulenchromatographie gereinigt wird.

**16.** Arzneimittel nach den vorhergehenden Ansprüchen, dadurch gekennzeichnet, daß es in einer Form vorliegt, die wasserlöslich ist und nach Auflösen des lyophilisierten Produkts in destilliertem Wasser für die Verabreichung auf intramuskulärem und intravenösem Wege geeignet ist.

**17.** Arzneimittel nach Anspruch 16, dadurch gekennzeichnet, daß es $5 \cdot 10^{-5}$ bis $10^{-4}$ g (50 bis 100 $\gamma$) der wirksamen Fraktion enthält.

**18.** Arzneimittel nach Ansprüchen 16 und 17 für die Verwendung bei der Behandlung der onkogenetischen Formen.

**19.** Arzneimittel nach jedem der Ansprüche 16 bis 18 für die Verwendung bei der Behandlung von infektiösen pathologischen Zuständen mikrobiellen und viralen Ursprungs.

**20.** Arzneimittel nach jedem der Ansprüche 16 bis 18 für die Verwendung bei der Behandlung der pathologischen Zustände, bei denen das immunodefensive System angegriffen ist.